# EUROPÄISCHE PATENTANMELDUNG

(11) **EP 0 908 717 A2**
(43) Veröffentlichungstag der Anmeldung: **14.04.1999**
(21) Anmeldenummer: 98810965.8
(22) Anmeldetag: 24.09.1998
(51) Int. Cl.: G01N 21/17

(54) **Photoakustische Messeinrichtung mit freifallender Probenflüssigkeit**

(30) Priorität: 09.10.1997 DE 19744500
(71) Anmelder: ABB RESEARCH LTD., 8050 Zürich (CH)
(72) Erfinder: Byatt, John Anthony, 5313 Klingnau (CH); Kleiner, Thomas, 5416 Kirchdorf (CH); Matter, Daniel, Dr., 5200 Brugg (CH); Spanner, Günter, Dr., 5404 Baden (CH)
(74) Vertreter: Lück, Gert, Dr.

(57) **Zusammenfassung**

Die vorliegende Erfindung hat eine photoakustische Durchfluss - Messzelle 1a, 1b zur Messung von Ölrückständen in Wasser zum Gegenstand. Das bekannte photoakustische Sensorprinzip beruht auf der Umwandlung optischer Energie in Schallenergie durch Lichtabsorption an Ölmolekülen in Wasser. Eine Messzelle 1a, 1b mit verschmutzungsfreier Einkoppeloptik 9, 10, 11, 21 wird erfindungsgemäss dadurch geschaffen, dass eine Freifallstrecke 3 zur berührungslosen Lichteinkopplung 12 in den Analyten 4 vorgesehen ist. Der Schalldetektor 16 ist ein aussenmontierter piezoelektrischer Aufnehmer 16 oder ein optisches Interferometer 23, das Schwingungen der Flüssigkeitsoberfläche 6 berührungsfrei misst. Die photoakustische Freifall - Messzelle 1a, 1b zeichnet sich durch hohe Nachweisstärke bis in den ppm - Konzentrationsbereich und geringe Störanfälligkeit aus. Sie ist speziell für den Einsatz in Hochdruck - Separatortanks 28 bei der Erdölförderung geeignet.

## Beschreibung

### TECHNISCHES GEBIET

Die Erfindung bezieht sich auf das Gebiet der photoakustischen Spektroskopie zur Detektion von Verunreinigungen in flüssigen Medien. Sie geht aus von einer photoakustischen Durchfluss - Messzelle nach dem Oberbegriff des Anspruchs 1.

### STAND DER TECHNIK

Bei der Offshore-Erdölförderung werden sog. Separationstanks eingesetzt, in welchen die bei der Bohrung bzw. Förderung auftretenden verschiedenen Phasen (Sand, Wasser, Öl und Gas) aufgrund ihrer Dichteunterschiede separiert und in getrennten Leitungssystemen abgeführt werden. Dabei können auch geringe Ölverunreinigungen im Abwasser zu intolerablen Umweltbelastungen mit entsprechenden Kostenfolgen führen. Typische Konzentrationsgrenzwerte für Öl in Wasser betragen bei direkter Entsorgung ins Meer 40 ppm und bei Wiederverwendung als Prozesswasser, beispielsweise beim Zurückpumpen in die Ölquelle, 100 ppm - 5000 ppm. Zur Überwachung der Grenzwerte sind daher nachweisempfindliche und zuverlässige Ölrückstandsdetektoren notwendig.

Neuerdings werden Hochdruck - Separationstanks entwickelt, die für den Betrieb auf dem Meeresboden einige 100 m unterhalb der Meeresoberfläche geeignet sind. Das geförderte und bereits separierte Öl kann dann mit viel geringerem Energieaufwand an die Meeresoberfläche gepumpt werden. Solche Separatortanks sind sehr hohen Drücken von 60 - 180 bar, nämlich von aussen dem Wasserdruck am Meeresgrund und von innen dem Druck des geförderten Erdöls, sowie hohen Temperaturen von 50 - 120 °C ausgesetzt. Ein Ölrückstandsdetektor muss unter diesen schwierigen Betriebsbedingungen jahrelang und wartungsfrei funktionsfähig sein, da ein Betriebsausfall und vorzeitiger Ersatz hohe Kosten verursachen würde.

Aus dem Artikel von H. A. MacKenzie et al., "A laser photoacoustic sensor for analyte detection in aqueous systems", Sensors and Actuators B, 11, 213 - 220 (1993) sind photoakustische Sensoren zur Spurenanalyse in Flüssigkeiten und speziell von Öl in Wasser bekannt. Das photoakustische Messverfahren beruht auf der Umwandlung optischer Energie in Schallenergie durch Fremdmoleküle in Flüssigkeiten. Intensive gepulste Laserstrahlung wird in eine Messzelle gesendet, von den zu detektierenden Molekülen absorbiert und durch strahlungslose Relaxation in thermische Energie umgewandelt. Die lokale Erwärmung im Absorptionsbereich führt zu thermischer Expansion und zur Abstrahlung einer Schalldruckwelle. Die Frequenz der Schallwelle wird durch die Repetitionsrate oder Modulationsfrequenz der Laserstrahlung bestimmt. Die Anregungseffizienz ist proportional zur absorbierten Laserpulsenergie, zum thermischen Expansionskoeffizienten, zur Schallgeschwindigkeit und umgekehrt proportional zur Wärmekapazität. Günstigerweise wird die Wellenlänge so gewählt, dass die Absorption im Medium gering und in den Fremdmolekülen hoch ist. Die Nachweisstärke für Verunreinigungen ist bei hohen Laserpulsenergien und in kurzen Messzellen um mehrere Grössenordnungen besser als bei direkter Infrarot - Transmissions- oder Remissionsspektroskopie.

In Messzellen für aggressive oder belagbildende Analyten stellt die Verschmutzung von Sensorkomponenten, wie z. B. der optischen Fenster oder auch der akustischen Detektoren, ein erhebliches Problem dar. Aus dem U. S. Pat. No. 5,125,749 ist eine photoakustische Durchfluss - Messzelle für Flüssigkeiten bekannt, bei der ein Sensorkopf mit einer faseroptischen Lichtzuführung, einem optischen Fenster, einem Schalldruckaufnehmer und einer elektrischen Leitung zur Signalübertragung an eine Auswerteelektronik ausgestattet ist. Der Sensorkopf und insbesondere das optisches Fenster ist dem Flüssigkeitsstrom unmittelbar ausgesetzt. Der Sensor ist deshalb sehr anfällig für Ablagerungen auf dem optischen Fenster. Für die Anwendung in einem Separatortank muss nämlich aufgrund der extremen und veränderlichen Temperatur- und Druckverhältnisse mit einer starken Belagsbildung durch öl- oder wachsähnlichen Schichten gerechnet werden.

In dem U. S. Pat. No. 5,339,674 wird eine photoakustische Durchfluss - Messzelle für chemisch aggressive Gase offenbart. Das optische Fenster und das Mikrophon sind durch ein längliches Rohr von der photoakustischen Wechselwirkungszone getrennt und zusätzlich durch ein inertes Schutzgas vor Kontamination mit dem Messgas geschützt. Das gasdurchströmte Rohr dient dabei als optische Übertragungstrecke und zugleich als akustischer Wellenleiter. Diese Messanordnung ist jedoch für Flüssigkeits - Messzellen wegen des akustischen Impedanzsprungs zwischen der Flüssigkeit und dem Schutzgas ungeeignet.

Im Stand der Technik werden auch optische Methoden zur berührungslosen Detektion von Ultraschallwellen an Oberflächen angegeben. Im Artikel von Jean - Pierre Monchalin, "Optical Detection of Ultrasound", IEEE Transactions on Ultrasonics, Ferroelectrics, and Frequency Control 33 (5), 485 - 499 (1986) werden optische Schalldetektoren beschrieben, die auf Strahlablenkung oder interferometrischer Interferenzbildung beruhen. Monolithisch aufgebaute, alltagstaugliche Interferometer mit integrierter Laser- und Photodiode sind heutzutage kommerziell erhältlich.

### DARSTELLUNG DER ERFINDUNG

Aufgabe der vorliegenden Erfindung ist es, eine verbesserte photoakustische Durchfluss - Messzelle für Flüssigkeiten anzugeben, bei der Probleme mit der Kontamination von Sensorkomponenten weitgehend vermieden werden und die sich durch eine grosse Messempfindlichkeit und Langzeitzuverlässigkeit auszeichnet. Diese Aufgabe wird erfindungsgemäss durch die Merkmale des Anspruchs 1 gelöst.

Kern der Erfindung ist es nämlich, eine optisch kontaktfreie photoakustische Flüssigkeits - Messzelle dadurch zu realisieren, dass die Messzelle eine Freifallstrecke für die Flüssigkeit aufweist.

Ein erstes Ausführungsbeispiel zeigt eine Durchfluss - Messzelle mit einem vertikal fallenden, zylindrischen Flüssigkeitsstrahl und einem horizontalen, blattförmigen Laserstrahl, der ebene Schallwellen in der Flüssigkeitssäule anregt, die durch einen piezoelektrischen Schalldruckaufnehmer aussen am Abflussrohr detektiert werden.

Ein zweites Ausführungsbeispiel stellt eine Variante der Freifall - Messzelle dar, bei welcher ein fokussierter Laserstrahl zylindrische Schallwellen in der Flüssigkeitssäule anregt und die resultierenden Oberflächenschwingungen der Flüssigkeitssäule völlig berührungsfrei mit einem optischen Interferometer gemessen wird.

Ein drittes Ausführungsbeispiel zeigt einen Einbau einer erfindungsgemässen photoakustischen Freifall - Messzelle zur Konzentrationsmessung von Öl in Wasser in einem Hochdruck - Separatortank.

Zusätzliche Ausführungsbeispiele ergeben sich aus den abhängigen Ansprüchen.

Ein wichtiger Vorteil der erfindungsgemässen photo akustischen Zelle ist ihre ausgezeichnete Eignung für Messungen an chemisch aggressiven oder belagbildenden Flüssigkeiten.

Ein weiterer Vorteil besteht darin, dass trotz räumlicher Trennung zwischen dem Analyten und den Sensorkomponenten eine gute optische und akustische Kopplung an den Analyten realisiert wird.

Desweiteren vorteilhaft sind der einfache, robuste Aufbau ohne bewegliche Teile, die weitgehende Wartungsfreiheit und die Möglichkeit zur in - situ Fernüberwachung flüssiger Analyten.

### KURZE BESCHREIBUNG DER ZEICHNUNG

Die Erfindung wird nachstehend anhand von Ausführungsbeispielen erläutert. Es zeigen:
- Fig. 1: eine erste erfindungsgemässe photoakustische Freifallzelle mit einem piezoelektrischen Schalldetektor;
- Fig. 2: eine Detailansicht der photoakustischen Wechselwirkungszone in einer Freifallzelle gemäss Fig. 1;
- Fig. 3: eine zweite erfindungsgemässe photoakustische Freifallzelle mit einem optischen Schalldetektor in Form eines monolithisch aufgebauten Interferometers;
- Fig. 4: eine Detailansicht der photoakustischen Wechselwirkungszone in einer Freifallzelle gemäss Fig. 3;
- Fig. 5: einen Einbau einer photoakustischen Freifallzelle in einem Hochdruck-Separatortank.
In den Figuren sind gleiche Teile mit gleichen Bezugszeichen versehen.

### WEGE ZUR AUSFÜHRUNG DER ERFINDUNG

Die Erfindung hat eine photoakustische Messzelle 1a, 1b für flüssige Analyten, insbesondere zur Konzentrationsmessung von Öl in Wasser, zum Gegenstand. Die photoakustische Messzelle 1a, 1b umfasst Mittel 2, 3, 7, 8, zur Lenkung des Flüssigkeitsdurchflusses 4, optische Komponenten zur Lichteinkopplung bzw. eine Einkoppeloptik 9, 10, 11, 21 und Mittel 16, 17, 18, 23, 24 zur Schalldetektion.

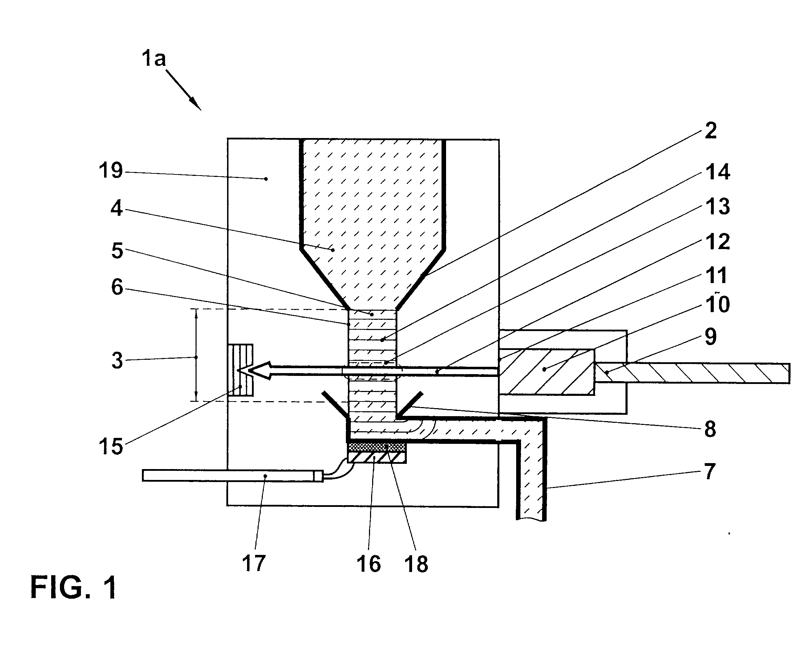

In Fig. 1 ist ein erstes Ausführungsbeispiel dargestellt. Die erfindungsgemässe Messzelle 1a weist eine Freifallstrecke 3 für die zu untersuchende Flüssigkeit 4 auf. Es ist erfindungswesentlich, dass der Flüssigkeitsstrahl 5 im Bereich der Freifallstrecke 3 nicht geführt ist, d. h. er ist ausschliesslich durch seine eigene Oberfläche 6 begrenzt. Hingegen besteht eine grosse Freiheit bezüglich der genauen Führung des Flüssigkeitsstrahls 5. Insbesondere kann eine Freifallstrekke 3 auch für einen horizontalen oder einen gekrümmten Strahl 5 ausgelegt sein. Vorzugsweise ist die Freifallstrecke 3 für einen zylindrischen Flüssigkeitsstrahl 5 ausgelegt, der insbesondere vertikal nach unten fallend ausgerichtet ist. Für diesen Zweck ist die Zuführung 2 für die Flüssigkeit 4 am Anfang bzw. oberhalb der Freifallstrecke 3 axialsymmetrisch und konisch zulaufend ausgebildet. Am Ende bzw. unterhalb der Freifallstrecke 3 ist ein Abflussrohr 7 angebracht, das vorzugsweise abgewinkelt und mit einem Auffangtrichter 8 versehen ist.

Die Messzelle 1a umfasst ferner eine Einkoppeloptik 9, 10, 11, insbesondere eine Lichtquelle 9 bzw. eine optische Zuführungsfaser 9, die mit einer Lichtquelle verbunden ist, und eine fokussierende Optik 10, beispielsweise eine Konvexlinse 10, die zur Bündelung des schallanregenden Lichtstrahls in der Freifallzelle 1a dient. Zum Schutz der Konvexlinse 10 kann die Freifallzelle 1a ein optisches Fenster 11 aufweisen. Mit Vorteil ist ein Schutzgas 19 zwischen der Freifallstrecke 3 und der Einkoppeloptik 9, 10, 11 vorgesehen. Durch die Einkoppeloptik 9, 10, 11 ist eine optische Achse bzw. eine Lichtstrecke 12 in der Messzelle 1a vorgegeben. Am Ende der Lichtstrecke 12 kann ein Strahlfänger 15 zur Vermeidung von Rückstreuung angebracht sein. Vorzugsweise ist die Lichtstrecke 12 senkrecht zur Freifallstrecke 3 ausgerichtet. Die Lichtstrecke 12 schneidet die Freifallstrecke 3 und definiert im Uberlappungsgebiet eine photoakustische Wechselwirkungszone 13. Zur Detektion der in der Zone 13 lichtinduzierten Schallwellen 14 ist die Freifallzelle 1a mit einem Schalldetektor 16 ausgerüstet. Grundsätzlich bestehen bezüglich des Typs und der räumlichen Anordnung des Schalldetektors 16 keine Einschränkungen. Der Schalldetektor 16 kann beispielsweise mit einem piezoelektrischen oder kapazitiven Schwingungsaufnehmer ausgerüstet und unterhalb oder oberhalb der Freifallstrecke 3, insbesondere an der Flüssigkeitszuführung 2 oder am Abflussrohr 7, montiert sein. Die Freifallzelle 1a ist über Leitungen 9, 17 mit einer nicht dargestellten Messapparatur verbunden.

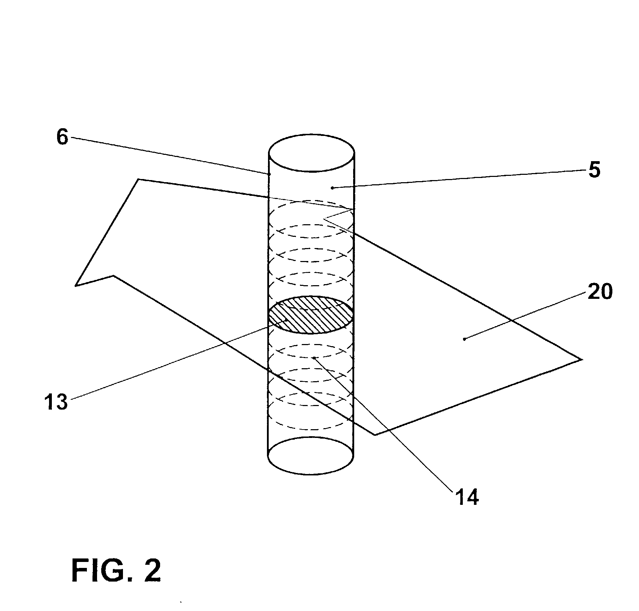

Eine bevorzugte erfindungsgemässe Ausgestaltung der photoakustischen Wechselwirkungszone 13 und des Schalldetektors 16 wird im Zusammenhang mit Fig. 2 erläutert. Mit Hilfe einer Zylinderoptik 10, insbesondere einer zylindrischen Konvexlinse 10, wird ein blattförmiger Laserstrahl 20 erzeugt, der sich in einer Ebene vorzugsweise senkrecht zur Achse der Freifallstrecke 3 bzw. des Flüssigkeitszylinders 5 ausbreitet. Im beleuchteten Querschnitt des Zylinders 5 werden photoakustisch ebene Schallwellen 14 erzeugt, die sich entlang der Zylinderachse bzw. der Freifallstrecke 3 beidseitig ausbreiten. Ein piezoelektrischer oder kapazitiver Schalldetektor 16 zum Nachweis der nach unten laufenden Welle ist aussen am Abflussrohr 7 befestigt. Für eine optimale Schallankopplung ist das Abflussrohr 7 unter 90° seitlich abgewinkelt und der Detektor 16 im Bereich der Abwinkelung unterhalb der Freifallstrecke 3 positioniert. Darüberhinaus kann eine Schicht 18 zur Schallimpedanzanpassung zwischen dem Detektor 16 und dem Abflussrohr 7 vorgesehen sein.

Ein piezoelektrischer Schallaufnehmer 16 kann aus einer piezoelektrischen Keramik, wie Bleizirkonate (PZT), oder aus einer elektrisch gepolten Folie, wie Polyvinyldifluorid (PVDF), Teflon oder Mylar, bestehen. Die Folie hat den Vorteil einer besseren Impedanzanpassung und eines kürzeren Nachschwingverhaltens. Kapazitive Schalldetektoren 16 sind zwar technisch etwas aufwendiger, aber sehr nachweisstark für Schallwellen.

Die erfindungsgemässe Anordnung des Schalldetektors 16 zeichnet sich durch eine grosse Messempfindlichkeit bei geringe Störanfälligkeit aus. Eine sehr gute Schallankopplung ist dadurch gewährleistet, dass die lichtinduzierten Ultraschallwellen mehrere Millimeter dicke Stahlwandungen und kalk- oder ölhaltige Innenwandbeläge ohne nennenswerte Abschwächung durchdringen und aufgrund der Geometrie des Abflussrohrs 7 sehr effizient empfangen werden. Die Aussenmontage am Abflussrohr 7 ist einfach und zuverlässig und schützt den Schalldetektor 16 mitsamt den elektrischen Signalleitungen 17 vor Korrosion.

Es ist auch ein Ziel der Erfindung, störende Ultraschallechos, z. B. aufgrund von Mehrfachreflexionen an der Flüssigkeitsoberfläche 6, klein zu halten. Die dafür erforderliche Planarität und vertikale Ausrichtung der Schallwellen 14 wird durch ein rechteckförmiges, breites und über den Zylinderquerschnitt homogenes Strahlprofil sowie durch eine senkrechte Einstrahlrichtung des Laserblatts 29 erreicht. Für diesen Zweck wird eine Zylinderoptik 10 mit einer horizontalen Strahlaufweitung grösser als der Offnungsdurchmesser der Zuführung 2 und einer deutlich kleineren vertikalen Strahlaufweitung gewählt. Das resultierende Laserblatt 20 ist dann breiter als der Zylinder 5 und in vertikaler Richtung scharf begrenzt.

Zur Verstärkung des photoakustischen Signals kann eine resonante Anregung von Längsschwingungen des Zylinders 5 vorgesehen sein. Dafür werden bei gegebener Schallgeschwindigkeit v die Zylinderlänge L und die Laserrepetitionsrate f aufeinander abgestimmt. Z. B. wird für v = 1500 m/s, L = 1,5 cm und f = 50 kHz eine longitudinale Zylinderschwingung angeregt.

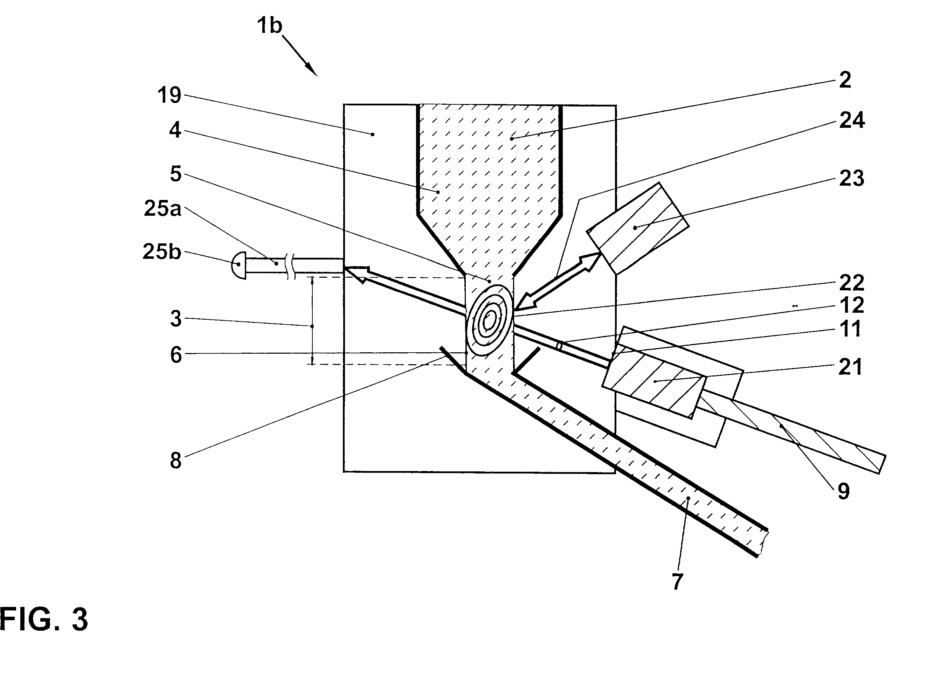

Die Fig. 3 zeigt eine zweite Ausführungsform einer Freifall - Messzelle 1b, welche erfindungsgemäss mit einem optischen Schalldetektor 23 ausgerüstet ist. Der sonstige Aufbau der Messzelle 1b, insbesondere die Anordnung der Freifallstrecke 3 und der Lichtstrecke 12, entspricht dem oben dargelegten Fall. Im Unterschied zur Messzelle 1a wird hier eine völlig berührungslose Schalldetektion realisiert. Das Messprinzip beruht darauf, dass die durch die Lichtquelle 9 photoakustisch induzierten Schallwellen 22 Oberflächenschwingungen des Flüssigkeitsstrahls 5 anregen, die mit einem optischen Abstandssensor bzw. Schalldetektor 23 der eingangs genannten Art nachweisbar sind. Der optische Schalldetektor 23 ist mit Sichtverbindung zur Freifallstrecke 3 angeordnet. Ein Detektionslichtstrahl 24 ist so auf die Freifallstrecke 3 ausgerichtet, dass genügend an der Oberfläche 6 rückreflektiertes oder rückgestreutes Licht vom Detektor 23 erfassbar ist.

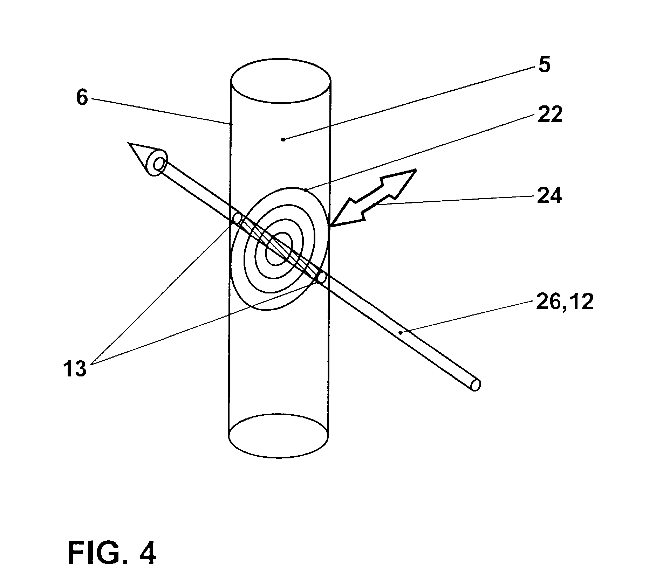

In Fig. 4 ist eine bevorzugte erfindungsgemässe Ausgestaltung der photoakustischen Wechselwirkungszone 13 dargestellt. Mit Hilfe einer sphärischen Einkoppeloptik 21, insbesondere einer sphärischen Konvexlinse 21, wird ein fokussierter gaussscher bzw. bleistiftförmiger Laserstrahl 26 erzeugt, der sich entlang der Lichtstrecke 12 ausbreitet. Die Lichtstrecke 12 ist wiederum vorzugsweise senkrecht zur Freifallstrecke 3 bzw. senkrecht zur Achse des Flüssigkeitszylinders 5 ausgerichtet. Im beleuchteten Teil des Zylinders 5 werden photoakustisch zylindrische Schallwellen 22 erzeugt, die sich von der Lichtstrecke 12 radial ausbreiten und die Oberfläche 6 in Schwingung versetzen. Grosse Schwingungsamplituden an der Oberfläche 6 werden dort auftreten, wo der Oberflächenvektor senkrecht zur Lichtstrecke 12 gerichtet ist. Deswegen ist der Detektionslichtstrahl 24 vorzugsweise parallel zu einem solchen Oberflächenvektor, d. h. senkrecht zur Lichtstrecke 12 und natürlich senkrecht zur Freifallstrecke 3, ausgerichtet.

Eine Signalverstärkung kann durch eine resonante akustische Anregung von Transversalschwingungen des Flüssigkeitsstrahls 5 erreicht werden. Für dünne Flüssigkeitsstrahlen 5 sind höhere Anregungsfrequenzen im Bereich einiger 100 kHz einfach erzielbar. Selbstverständlich sind auch hier beliebige Querschnitte des Flüssigkeitsstrahls 5 durch eine entsprechende Formgebung der Flüssigkeitszuführung 2 der Freifallstrecke 3 wählbar. Insbesondere kann die Zuführung 2 und damit die Flüssigkeitssäule 5 einen rechteckigen oder konkaven Querschnitt aufweisen. Eine konkave Oberfläche 6 ist beispielsweise zur Erzielung starker akustischer Resonanzen oder zur optischen Bündelung des rückreflektierten Detektionslichtstrahls 24 geeignet.

Bevorzugt ist der Schalldetektor 23 als monolithisches Interferometer 23, insbesondere als modulierbares Michelson - Interferometer mit integrierter Laserund Photodiode, ausgelegt. Dann befinden sich der Sendeausgang und der Empfangseingang des Schalldetektors 23 am gleichen Ort und der Detektionslichtstrahl 24 ist vorzugsweise senkrecht zur Oberfläche 6 orientiert, um die Intensität des in den Detektor 23 rückreflektierten Lichts zu maximieren. Auch können optische Fasern zur Ankopplung des optischen Schalldetektors bzw. Interferometers 23 an die Messzelle 1b vorgesehen sein.

Der Flüssigkeitsstrahl 5 ist häufig Erschütterungen und anderen störenden akustischen Anregungen unterworfen, die im Interferometersignal eliminert werden sollen. Für diesen Zweck ist das Michelson - Interferometer 23 auf einen fixen Arbeitspunkt geregelt, so dass niederfrequente Störungen und Drift im Sensorsignal ausgeglichen werden. Die hochfrequenten photoakustischen Schwingungen sind dann störungsfrei mit grosser Empfindlichkeit messbar.

Ultraschallechos durch Reflexionen an der Flüssigkeitsoberfläche 6 sind in der Freifallzelle 1b gemäss Fig. 3 und 4 unvermeidlich. Die Bandbreite des optischen Detektors 23 ist mit Vorteil so gross zu wählen, dass das primäre photoakustische Signal von den Echos diskriminierbar ist. Beispielsweise beträgt für einen Durchmesser des Flüssigkeitsstrahls 5 von 10 mm der Zeitabstand der Echos 1 µs und die erforderliche Bandbreite einige MHz.

Die Überlegungen zur Wahl der Lichtquelle 9 und des Schutzgases 19 sind für beide Varianten der Freifallzelle 1a, 1b die gleichen. Die Lichtquelle ist vorzugsweise ein Halbleiterlaser und insbesondere eine Laserdiode. Laserdioden sind leistungsstark, langlebig, druckunempfindlich, im gewünschten nahinfraroten Spektralbereich (z. B. 0.7 µm - 2.5 µm) kommerziell erhältlich und einfach über Lichtwellenleiter anschliessbar. Bevorzugt werden gepulste Laserdioden verwendet, deren Anregungsfrequenzen von typischerweise 100 kHz - 10 Mhz ausserhalb des akustischen Störspektrums liegen. Bis ca. 10 kHz sind auch modulierte Dauerstrich - Laserdioden einsetzbar. Schwankungen der Laserleistung können erfindungsgemäss überwacht und korrigiert werden, indem die durch die Freifallzelle 1a, 1b transmittierte Lichtleistung gemessen wird. Dafür ist die Messzelle 1a, 1b an einem Ende der Lichtstrecke 12 statt mit dem Strahlfänger 15 beispielsweise über eine optische Faser 25a mit einem optischen Referenzdetektor 25b verbunden, der ein zur transmittierten Lichtleistung proportionales Signal bildet.

Durch die Wahl der Wellenlänge können die Messempfindlichkeit und der Messbereich variiert werden. Zur Konzentrationsbestimmung von Öl in Wasser mit hoher Nachweisstärke werden Wellenlängen nahe bei den Absorptionsmaxima der Öle oder Ölbestandteile gewählt. Jedoch ist dann der Dynamikbereich durch einen Sättigungseffekt bei höheren Konzentrationen eingeschränkt. Für Messungen mit grossem Dynamikbereich werden Wellenlängen in Spektralgebieten mit nur relativ schwachen Olabsorptionsbanden gewählt. In jedem Fall soll die Hintergrundabsorption im Wasser klein sein. Günstigerweise ist die Wellenlänge der Laserdiode 9 im Spektralbereich zwischen zwei Wasserabsorptionsbanden, beispielsweise zwischen 1,48 µm und 1,93 µm, gewählt.

Das Schutzgas 19 verhindert eine Kontamination und Schädigung der Einkoppeloptik 9, 10, 11. Zu diesem Zweck ist es chemisch inert und steht unter hinreichend hohem Druck. Das Schutzgas 19 kann in der Freifallzelle 1a stationär gefangen sein oder über nicht dargestellte Zu- und Ableitungen zirkulieren. Photoakustische Wechselwirkungen im Schutzgas 19 sind unbedenklich. Der Impedanzsprung zwischen dem Gas 19 und dem Flüssigkeitsstrahl 5 ist nämlich so gross, da die Schalltransmission vom Gas 19 in den Flüssigkeitsstrahl 5 und von dort zu einem Schalldetektor 16, 23 gering ist. Insbesondere kann als Schutzgas 19 anstelle von beispielsweise Luft, Stickstoff oder Argon auch das bei der Ölförderung anfallende Erdgas verwendet werden.

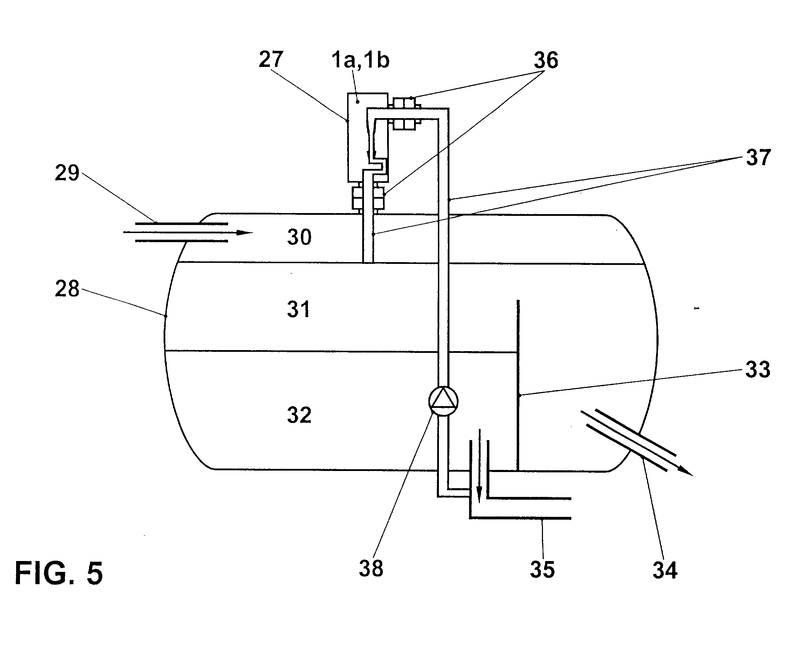

In Fig. 5 ist eine erfindungsgemässe Montage der Freifallzelle 1a, 1b auf einem Hochdruck - Separatortank 28 zu sehen. Der Tank 28 wird über einen Einlass 29 befüllt. Durch Sedimentation werden die Phasen Gas 30, Öl 31 und Wasser 32 getrennt. Das Öl 31 wird durch einen Auslass 34 und das Wasser 32 durch einen Auslass 35 abgelassen. Die Freifallzelle 1a, 1b befindet sich in einem Druckgehäuse 27, das über Anschlüsse 36 und Zuleitungen 37 mit dem Wasserauslass 35 und dem Tank 28 in Verbindung steht. Die elektrischen 17 und/oder faseroptischen 9, 25a Leitungen und die Messaparatur sind nicht dargestellt. Der Durchfluss 4 in der Messzelle 1a, 1b ist mit einer Pumpe 38 regelbar. Der Innendruck der Zelle 1a, 1b entspricht dem Druck im Separatortank 28. Das Schutzgasvolumen 19 in der Freifallzelle 1a, 1b kann durch nicht explizit dargestellte Leitungen mit dem Gas 30 im Tank 28 in Austausch stehen.

Die erfindungsgemässe Freifallzelle 1a, 1b hat erhebliche Vorteile gegenüber dem Stand der Technik. Durch die berührungsfreie optische Anregung wird ein direkter Kontakt zwischen der Einkoppeloptik 9, 10, 11 und dem Analyten 4, 5, 6 vermieden. Auf Vorrichtungen zur Reinigung eines optischen Fensters 11 und auf das Fenster 11 selber kann verzichtet werden. Die Schalldetektoren 16, 23 sind sehr messempfindlich und vor dem Analyten 4, 5, 6 gut geschützt. Mit dem optischen Schalldetektor 23 ist eine rein faseroptisch angesteuerte, galvanisch getrennte Freifallzelle 1b ohne aktive optische oder elektrische Komponenten realisierbar. Eine solche Freifallzelle 1b ist für die in - situ Fernüberwachung aggressiver oder kontaminierender Analyten in Umgebungen mit hohen Temperaturen, hohen Drücken und starken elektromagnetischen Störungen sehr geeignet.

### BEZUGSZEICHENLISTE

- 1a, 1b: photoakustische Freifall - Messzellen
- 2: Flüssigkeitszuführung
- 3: Freifallstrecke
- 4: Flüssigkeit, Analyt
- 5: Flüssigkeitsstrahl
- 6: Oberfläche des Flüssigkeitsstrahls
- 7: Abflussrohr
- 8: Auffangtrichter
- 9: Lichtquelle, Laserdiode, optische Zuführungsfaser
- 10: zylindrische Konvexlinse
- 11: optisches Fenster
- 12: Lichtstrecke
- 13: photoakustisch Wechselwirkungszone
- 14: ebene Schallwellen
- 15: Strahlfänger
- 16: piezoelektrischer Schalldetektor, Mikrophon
- 17: elektrische Signalleitung
- 18: Schicht zur Schallimpedanzanpassung
- 19: Schutzgas
- 20: Laserblatt
- 21: spärische Konvexlinse
- 22: zylindrische Schallwellen
- 23: optischer Schalldetektor, monolithisches Interferometer
- 24: Detektionslichtstrahl
- 25a: optische Faser
- 25b: optischer Referenzdetektor
- 26: bleistiftförmiger Laserstrahl
- 27: Druckgehäuse
- 28: Separatortank
- 29: Einlass
- 30: Gas
- 31: Öl
- 32: Wasser
- 33: Trennwand
- 34: Ölauslass
- 35: Wasserauslass
- 36: Anschlüsse
- 37: Zuleitungen
- 38: Pumpe

## Patentansprüche

1. Photoakustische Durchfluss - Messzelle (1a, 1b) für Flüssigkeiten (4), insbesondere geeignet zur Konzentrationsmessung von Öl in Wasser, dadurch gekennzeichnet, dass die Messzelle (1a, 1b) eine Freifallstrecke (3) für die Flüssigkeit (4) aufweist.

2. Photoakustische Durchfluss - Messzelle (1a, 1b) nach Anspruch 1, dadurch gekennzeichnet, dass
a) die Freifallstrecke (3) für einen zylindrischen, vertikal fallenden Flüssigkeitsstrahl (5) ausgelegt ist und
b) ein Schutzgas (16) zwischen der Freifallstrecke (3) und einer Einkoppeloptik (9, 10, 11, 21) vorgesehen ist.

3. Photoakustische Durchfluss - Messzelle (1a, 1b) nach Anspruch 2, dadurch gekennzeichnet, dass
a) die Einkoppeloptik (9, 10, 21) eine optische Zuführungsfaser (9) und eine Konvexlinse (10, 21) umfasst,
b) die optische Zuführungsfaser (9) mit einer gepulsten Laserdiode verbunden ist und
c) eine durch die Einkoppeloptik (9, 10, 21) vorgegebene Lichtstrecke (12) senkrecht zur Freifallstrecke (3) ausgerichtet ist.

4. Photoakustische Durchfluss - Messzelle (1a) nach Anspruch 3, dadurch gekennzeichnet, dass
a) die Konvexlinse (10) zylindrisch ist,
b) an einem Ende der Freifallstrecke (3) ein abgewinkeltes Abflussrohr (7) angebracht ist und
c) ein piezoelektrischer oder kapazitiver Schalldetektor (16) aussen am Abflussrohr (7) befestigt ist.

5. Photoakustische Durchfluss - Messzelle (1b) nach Anspruch 3, dadurch gekennzeichnet, dass
a) die Konvexlinse (21) sphärisch ist und
b) die Messzelle (1b) mit einem optischen Schalldetektor (23) ausgerüstet ist.

6. Photoakustische Durchfluss - Messzelle (1b) nach Anspruch 5, dadurch gekennzeichnet, dass
a) der optische Schalldetektor ein Interferometer (23) ist und
b) ein Detektionslichtstrahl (24) des Interferometers (23) senkrecht zur Lichtstrecke (12) und senkrecht zur Freifallstrecke (3) ausgerichtet ist.

7. Photoakustische Durchfluss - Messzelle (1b) nach Anspruch 6, dadurch gekennzeichnet, dass optische Fasern zur Ankopplung des Interferometers (23) an die Messzelle (1b) vorgesehen sind.

8. Photoakustische Durchfluss - Messzelle (1a, 1b) nach einem der Ansprüche 1 - 7, dadurch gekennzeichnet, dass
a) die Messzelle (1a, 1b) an einem Ende einer Lichtstrecke (12) mit einem optischen Referenzdetektor (25b) verbunden ist und
b) ein Druckgehäuse (27) mit Anschlüssen (36) für Zuleitungen (37) zur Montage der Messzelle (1a, 1b) auf einem Separatortank (28) vorgesehen ist.
